# FASCICULE DE BREVET EUROPEEN

(11) **EP 1 381 414 B1**
(45) Date de publication et mention de la délivrance du brevet: **07.09.2005**
(21) Numéro de dépôt: 02714278.5
(22) Date de dépôt: 05.03.2002
(51) Int. Cl.: A61M 5/32, A61M 5/31

(54) **SERINGUE**
SPRITZE
SYRINGE

(30) Priorité: 05.03.2001 FR 0102984
(43) Date de publication de la demande: 21.01.2004
(73) Titulaire: Becton Dickinson France, 38800 Le Pont-de-Claix (FR)
(72) Inventeur: BARRELLE, Laurent, F-38250 Saint Nizier du Moucherotte (FR); JANSEN, Hubert, F-38560 Haute Jarrie (FR)
(74) Mandataire: Brédeville, Odile Marie
(86) Numéro de dépôt international: PCT/FR2002/000792
(87) Numéro de publication internationale: WO 2002/070055

(56) Documents cités:
- EP-A- 0 864 335
- EP-A- 1 062 961
- US-A- 3 921 633
- US-A- 5 242 416
- US-A- 5 509 903
- US-A- 5 554 133
- US-A- 5 700 247

## Description

La présente invention concerne une seringue, ou dispositif similaire.

Une seringue comprend parfois un corps de seringue devant être relié, du côté proximal, à une bague.

Les bagues existantes présentent toutefois l'inconvénient principal de ne pas pouvoir recevoir des corps de seringue de différents diamètres. Or, des corps de seringue de diamètres différents sont utilisés en fonction des besoins, et ces seringues existantes ne peuvent donc être adaptées à ces besoins spécifiques.

Ces seringues existantes présentent également différents inconvénients liés soit à leur relative complexité de structure, soit à des problèmes de fabrication résultant des tolérances dimensionnelles de leurs pièces constitutives ou des tolérances d'assemblage de ces pièces, soit à l'existence de jeux entre lesdites pièces.

US 5, 242, 416 décrit un dispositif de protection d'une seringue, ledit dispositif comprenant un fourreau pour recevoir une partie de la seringue.

EP 0 864 335 décrit un dispositif de protection d'une ampoule, comprenant un corps recevant ladite ampoule et un fourreau protecteur monté coulissant sur ledit corps.

La présente invention vise à remédier à l'ensemble de ces inconvénients.

Son objectif principal est donc de fournir une seringue pouvant recevoir des corps de seringue de différents diamètres, de sorte que cette seringue puisse comprendre des pièces standardisées et recevoir des corps de seringue différents, en fonction des besoins.

Un autre objectif de l'invention est de parvenir à l'objectif indiqué ci-dessus sans complexifier outre mesure la structure ou l'assemblage de la seringue, voire même en simplifiant cette structure et cet assemblage par rapport aux seringues existantes.

La seringue concernée comprend un corps de seringue et une bague à laquelle le corps de seringue est relié du côté proximal.

Selon l'invention,
- la bague comporte au moins deux parties de maintien, par exemple diamétralement opposées, destinées à recevoir et à maintenir entre elles le corps de seringue ; chaque partie de maintien présente une forme de fourche, c'est-à-dire comprend une paire de bras et un corps de liaison reliant ces bras à la bague, les bras de chaque partie de maintien étant conformés de manière à pouvoir entourer partiellement le corps de seringue ; et
- lesdits bras sont réalisés en un matériau présentant un degré de souplesse élastique tel qu'ils peuvent être déformés élastiquement de manière
à pouvoir recevoir entre eux, et enserrer, des corps de seringue de diamètres extérieurs différents.

La seringue selon l'invention peut ainsi comprendre une bague standardisée susceptible de recevoir des corps de seringue de différents diamètres extérieurs, en fonction des besoins.

La seringue peut notamment comprendre deux parties de maintien, diamétralement opposées.

Chacun des bras d'une partie de maintien peut comprendre au moins un bossage faisant saillie radialement vers l'intérieur, pour assurer l'immobilisation, et éventuellement le centrage, d'un corps de seringue d'un diamètre réduit.

Un chanfrein peut être aménagé dans lesdits bras du côté de la bague par lequel un corps de seringue est destiné à être introduit au travers de cette bague.

Ce chanfrein permet ainsi de faciliter l'engagement d'un corps de seringue entre lesdits bras.

Avantageusement, la seringue comprend :
- un étui de protection, engagé autour du corps de seringue, cet étui et ce corps étant mobiles l'un par rapport à l'autre entre une position d'utilisation du dispositif, dans laquelle l'aiguille de la seringue est exposée, et une position de sécurité, dans laquelle cette aiguille est entourée par l'étui de manière à prévenir tout risque de piqûre ou de coupure, et donc de contamination éventuelle, de l'utilisateur, après utilisation ; l'étui de protection comprend au moins une lumière ou saignée longitudinale ;
- la bague forme un moyen de déplacement du corps de seringue et de l'étui de protection l'un par rapport à l'autre entre lesdites positions d'utilisation et de sécurité, et présente à cet effet une partie logée à l'intérieur de l'étui de protection, comportant lesdites parties de maintien, et au moins une partie de prise d'appui, engagée au travers de ladite lumière ou saignée et faisant saillie au-delà de l'étui de protection, qui sert à la prise d'appui de l'index ou du majeur de la main de l'utilisateur;
- la tige du piston de la seringue comprend deux parties télescopiques, dont une, distale, est reliée au piston de la seringue, et dont l'autre, proximale, comporte une tête formant une partie de prise d'appui pour le pouce de l'utilisateur ; et
- au moins un pont ruptible relie les deux parties télescopiques de la tige de piston, ce ou ces ponts étant propres à se rompre au-delà d'un seuil de forces antagonistes exercées sur ladite partie de prise d'appui et ladite tête, ce seuil étant supérieur à la force nécessaire au déplacement du piston mais inférieur aux forces antagonistes qu'un utilisateur est susceptible d'exercer manuellement sur ladite partie de prise d'appui et ladite tête.

La bague forme ainsi un moyen de déplacement du corps et de l'étui l'un par rapport à l'autre entre lesdites positions d'utilisation et de sécurité. L'action permettant d'amener le corps et l'étui en position de sécurité est réalisée par la même prise manuelle que la prise par laquelle la seringue est maintenue au cours de son utilisation, et peut donc être réalisée dans le prolongement de cette utilisation.

La rupture du ou des ponts ruptibles génére un mouvement relatif brusque du corps de seringue et de l'étui de protection, tel que ce corps de seringue et cet étui sont amenés en position de sécurité à l'issue de ce mouvement brusque. Ce mouvement limite ou même interdit tout contrôle intempestif du déplacement relatif du corps et de l'étui.

Avantageusement, l'étui de protection présente des moyens de calage permettant de caler latéralement un corps de seringue mis en place dans cet étui, ces moyens de calage étant mobiles radialement de manière à pouvoir caler des corps de seringue de diamètres extérieurs différents.

Ces moyens de calage peuvent en particulier être aménagés du côté distal de l'étui de protection, pour caler latéralement la partie distale d'un corps de seringue.

Selon une forme de réalisation préférée de ces moyens de calage, l'étui de protection présente au moins deux fentes en forme de "U" diamétralement opposées, qui individualisent chacune une patte mobile radialement, la face interne de chacune de ces pattes présentant, du côté de l'extrémité de la patte non reliée à l'étui de protection, un bossage faisant saillie à l'intérieur de cet étui.

Le corps de seringue vient en appui contre ces bossages lors de sa mise en place dans l'étui de protection. Dans le cas de corps de seringue de plus grands diamètres extérieurs susceptibles d'être utilisés avec la seringue selon l'invention, les pattes se déforment radialement vers l'extérieur de manière à permettre l'effacement adéquat desdits bossages.

Avantageusement, le corps de liaison que comprend chaque partie de maintien est réalisé en un matériau présentant un degré de souplesse élastique, de telle sorte que ce corps de liaison présente une souplesse dans le sens radial de la bague, cette souplesse étant telle qu'elle permet de placer les bras de la partie de maintien selon plusieurs positions radiales distinctes.

Cette souplesse contribue ainsi à élargir la gamme de diamètres de corps de seringue pouvant être reçus par la bague, et à assurer un parfait maintien et un parfait centrage d'un corps de seringue d'un diamètre déterminé.

Avantageusement, chaque corps de liaison présente une forme coudée, c'est-à-dire comprend une partie intermédiaire, reliée auxdits bras et disposée sensiblement perpendiculairement à l'axe de la bague, et une partie de base, reliée à la bague et disposée sensiblement parallèlement à l'axe de cette bague.

Avantageusement,
- la bague comprend deux parties de prise d'appui telles que précitées, diamétralement opposées ;
- le corps de liaison de l'une des parties de maintien est reliée à la bague à proximité de l'une de ces saillies de prise d'appui et le corps de liaison de l'autre partie de maintien est relié à la bague à proximité de l'autre de ces saillies, et
- au moins une partie de ces corps de liaison est conformée de manière à faire saillie au-delà de la paroi de l'étui de protection.

La présence de ces corps de liaison permet ainsi d'écarter les doigts de l'utilisateur par rapport à la paroi de l'étui de protection, afin d'empêcher que ces doigts viennent frotter contre l'étui de protection lors du mouvement brusque précité, et qu'ils viennent ainsi contrarier ce mouvement.

Avantageusement :
- le corps de seringue présente une collerette proximale pour son assemblage à la bague ; et
- la bague comprend des moyens d'assemblage pour permettre son assemblage à cette collerette ; ces moyens d'assemblage sont constitués par deux parties d'assemblage de forme courbe, disposées de manière à former substantiellement un anneau ; ces parties d'assemblage sont reliées à la bague au niveau de zones périphériques et sont réalisées en un matériau présentant un degré de souplesse élastique ; les zones périphériques de ces parties d'assemblage sont situées l'une par rapport à l'autre à une distance supérieure au diamètre externe de ladite collerette tandis que les zones médianes de ces parties d'assemblage sont situées l'une par rapport à l'autre, à l'état non déformé de ces parties d'assemblage, à une distance inférieure au diamètre extérieur de la collerette ; ces zones médianes présentent, du côté de la bague par lequel le corps de seringue est engagé au travers des parties d'assemblage, des chanfreins conformés pour permettre l'effacement radial de ces zones médianes au moment de cet engagement.

L'engagement de la collerette entre ces parties d'assemblage provoque la déformation élastique de celles-ci au niveau desdites zones médianes, dont les chanfreins permettent l'effacement radial. Une fois la collerette engagée au-delà de ces parties d'assemblage, le rappel élastique du matériau constituant ces parties d'assemblage réalise un verrouillage du corps de seringue.

De préférence,
- la bague comprend deux parties de maintien telles que précitées, aménagées à un niveau distal par rapport auxdites parties d'assemblage ; et
- au moins une desdites parties de maintien présente une structure flexible, notamment au niveau de son corps de liaison, et est disposée de telle sorte que la distance séparant ses bras, d'une part, et lesdites parties d'assemblage, d'autre part, soit inférieure à l'épaisseur de la collerette.

Cette ou ces parties de maintien sont ainsi déformées lorsque la collerette est engagée entre elles et lesdites parties d'assemblage, et permettent de maintenir cette collerette plaquée contre ces parties d'assemblage. Une parfaite immobilisation axiale du corps de seringue est ainsi obtenue.

Pour sa bonne compréhension, l'invention est à nouveau décrite ci-dessous en référence au dessin schématique annexé représentant, à titre d'exemple non limitatif, une forme de réalisation préférée de la seringue qu'elle concerne.
La figure 1 en est une vue en perspective, sous forme d'une seringue, avant injection du produit que contient cette seringue ;
la figure 2 en est une vue de côté, en coupe longitudinale ;
la figure 3 est une vue à échelle agrandie, d'une portion de la tige de piston que comprend la seringue ;
la figure 4 est une vue de détail d'un étui de protection que comprend la seringue, à échelle agrandie et en coupe longitudinale ;
la figure 5 est une vue de côté, à échelle agrandie, de cet étui et d'une bague d'actionnement que comprend la seringue ;
la figure 6 est une vue axiale de cet étui et de cette bague, du côté proximal de la seringue ;
la figure 7 est une vue en perspective de cette même bague ;
la figure 8 est une vue axiale de cette bague, et d'un corps de seringue assemblé à elle, du côté distal de la seringue ;
la figure 9 est une vue similaire à la figure 8, après mise en place dans la bague d'un corps de seringue de plus gros diamètre que le corps de seringue montré sur la figure 8 ;
la figure 10 est une vue de la seringue similaire à la figure 2, une fois l'injection réalisée ; et
la figure 11 est une vue en coupe, à échelle agrandie, de la partie proximale de la seringue, une fois l'injection réalisée.

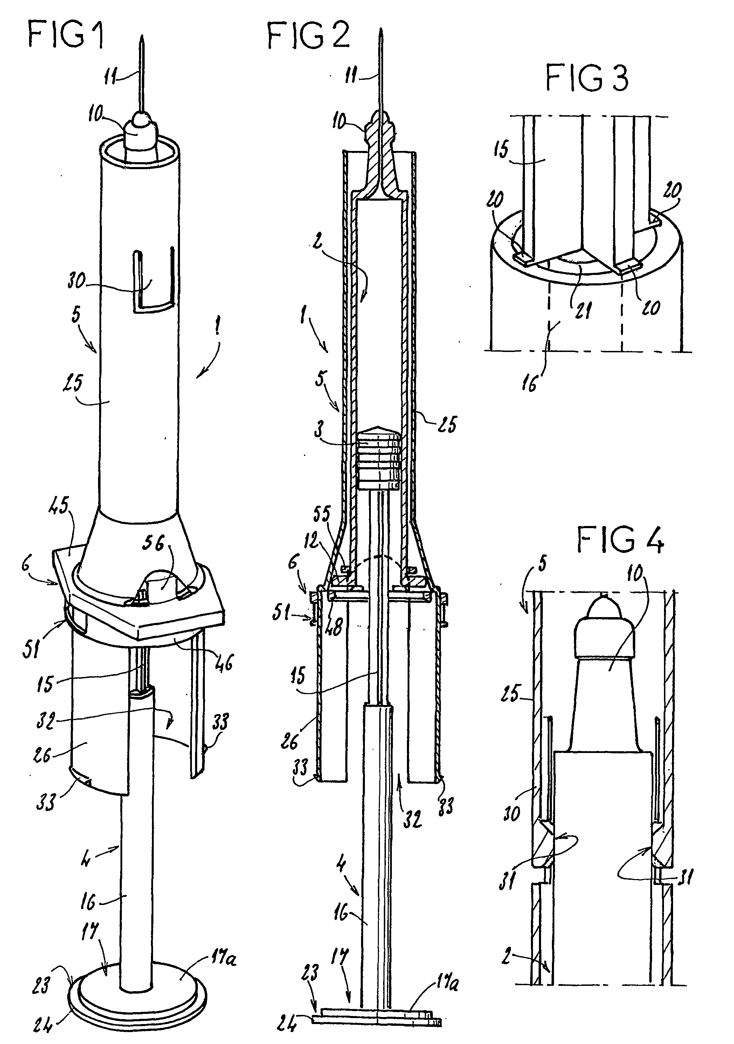

Les figures 1 et 2 représentent une seringue 1 à sécurité renforcée, comprenant un corps de seringue 2, un piston 3, une tige de piston 4, un étui de protection 5 et une bague 6 d'actionnement de l'étui 5.

Le corps de seringue 2 est de type classique, réalisé notamment en verre. Il comprend un embout distal 10 comportant l'aiguille d'injection 11 et une collerette proximale 12, faisant saillie radialement vers l'extérieur, qui permet l'assemblage du corps 2 avec la bague 6, comme cela sera décrit plus loin. Le montage de l'aiguille 11 peut être réalisé autrement qu'au moyen d'un tel embout 10.

Le piston 3 est de type classique.

La tige de piston 4 comprend deux parties télescopiques 15, 16, dont celle distale 15 présente une section transversale en forme de croix et dont celle proximale 16 présente une forme tubulaire, propre à recevoir la partie 15 à coulissement. La partie 15 peut avoir une section d'une autre forme qu'en croix, ou la partie 15 peut présenter une forme tubulaire et la partie 16 présenter une section transversale en forme de croix.

La partie 16 comprend une tête d'appui 17 pour le pouce de l'utilisateur.

Comme le montre plus particulièrement la figure 3, chacune des quatre ailes que présente la partie 15 est reliée à la partie 16 par au moins un pont de matière 20, de sorte que ces parties 15 et 16 sont normalement maintenues dans une position d'extension, montrée sur les figures 1 et 2.

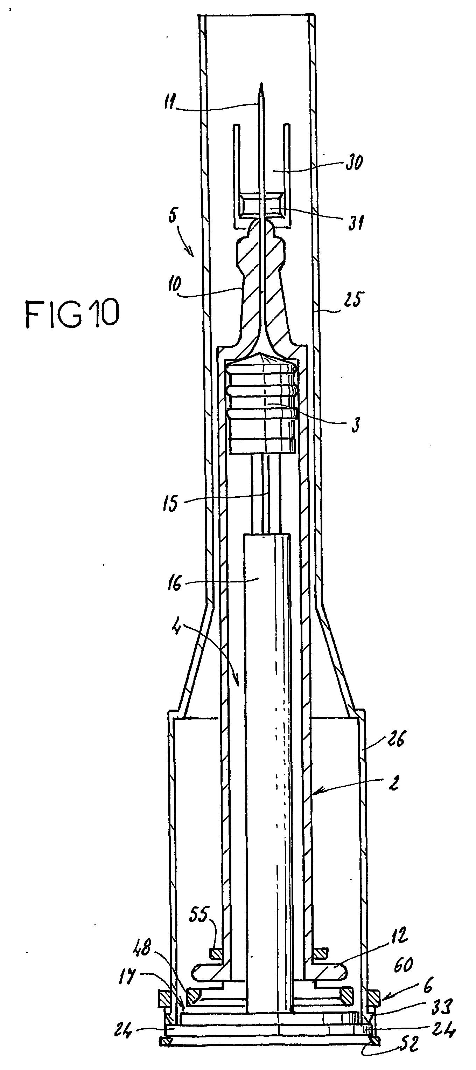

Ces ponts 20 sont suffisamment solides pour résister à la force qu'il est nécessaire d'exercer sur la tige 4 pour faire coulisser le piston 3 dans le corps 2, mais sont propres à se rompre au-delà d'un seuil de forces antagonistes susceptibles d'être exercées sur la bague 6 par l'index et le majeur de l'utilisateur, d'une part, et sur la tête 17 par le pouce de l'utilisateur, d'autre part, selon le geste classique d'actionnement d'une seringue. Une fois ces ponts 20 rompus, la partie 15 peut coulisser dans la partie 16 jusqu'à la position montrée sur la figure 10.

La partie 15 se prolonge, du côté proximal, au-delà des ponts 20, et cette prolongation 21 présente un diamètre extérieur légèrement inférieur au diamètre intérieur de la partie 16. Cette prolongation 21 permet de rigidifier longitudinalement la tige 4 afin d'éviter que cette tige présente, avant rupture des ponts 20, une zone de faiblesse dans le sens transversal.

La tête 17 présente une forme circulaire et comprend un redan périphérique 23, qui délimite une portion cylindrique 17a de la tête 17 et un rebord 24 faisant saillie radialement vers l'extérieur. Comme le montre la figure 11, le diamètre de cette portion cylindrique 17a est tel que cette portion cylindrique 17a peut être engagée avec ajustement dans la portion proximale 26 de l'étui 5 en fin d'injection.

L'étui 5 présente une portion distale 25 de diamètre interne supérieur au diamètre externe du corps 2 et une portion proximale 26 recevant la bague 6 à coulissement. L'ensemble de cet étui 5 est réalisé en une pièce par moulage d'un matériau synthétique présentant un degré de souplesse élastique telle qu'une matière plastique courante.

Comme le montrent les figures 1 et 4, la portion 25 présente deux fentes en forme de "U" diamétralement opposées, qui individualisent chacune une patte 30, cette patte 30 étant mobile radialement par déformation élastique au niveau de sa base. La face interne de chacune de ces pattes présente, du côté de l'extrémité de la patte 30 non reliée à l'étui 5, un bossage 31 faisant saillie à l'intérieur de cet étui.

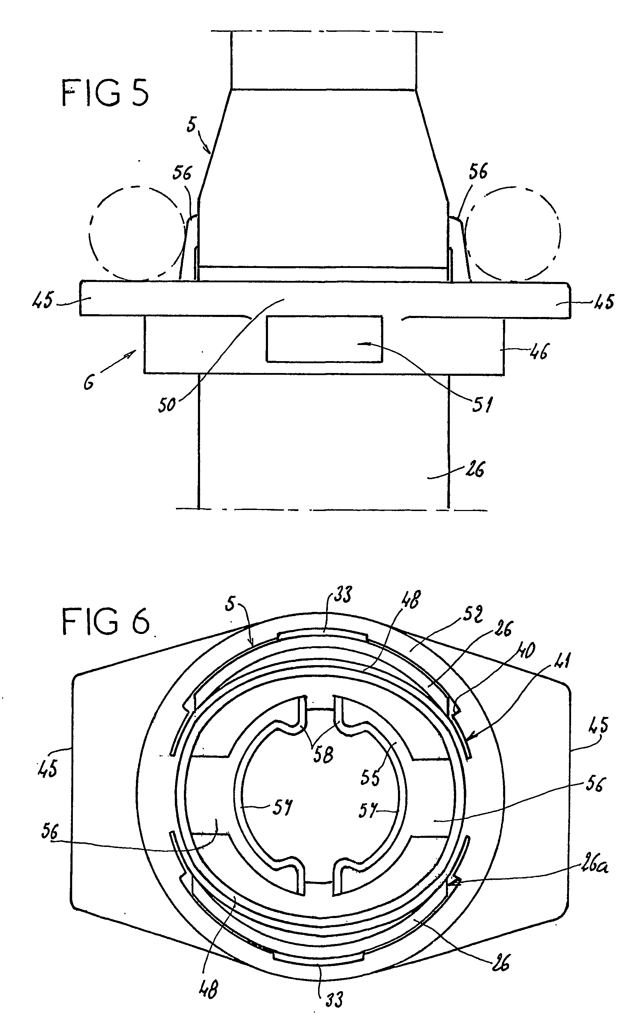

La portion 26 de l'étui présente deux méplats longitudinaux 26a (cf. figure 6) diamétralement opposés, au niveau desquels sont aménagés deux saignées 32 débouchant dans l'extrémité proximale de l'étui 5. Ces saignées 32 sont destinées à recevoir à coulissement des parties 56 de la bague 6, décrites plus loin.

Cette portion 26 présente en outre deux dents d'encliquetage 33 au niveau de son bord d'extrémité, à même d'assurer la rétention de la bague 6 sur l'étui 5, ainsi que cela sera également décrit plus loin.

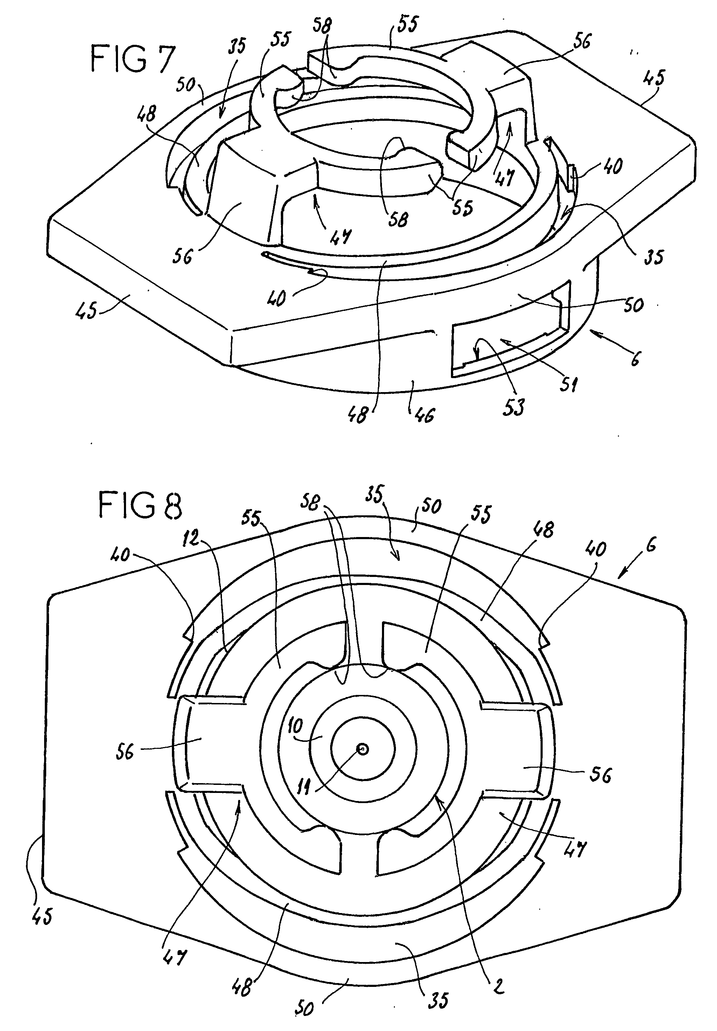

En référence à la figure 7, il apparaît que la bague 6 comprend deux lumières 35 de forme arquée, disposées symétriquement par rapport à l'axe de cette bague. Comme cela est visible sur la figure 6, ces lumières 35 sont destinées à recevoir les parties de l'étui 5 s'étendant entre lesdites saignées 32.

En retrait des extrémités longitudinales de ces lumières 35, sont formés des épaulements de calage 40, permettant de caler en rotation la bague 6 par rapport à l'étui 5. Chaque lumière 35 se prolonge au-delà de chaque épaulement 40, sous forme d'une fente 41.

La bague 6 présente deux ailettes d'appui 45 diamétralement opposées, faisant saillie radialement vers l'extérieur, une jupe proximale 46, deux parties de maintien 47 et deux parties d'assemblage 48. L'ensemble de cette bague 6 est réalisé en une pièce par moulage d'un matériau synthétique présentant un degré de souplesse élastique telle qu'une matière plastique courante.

Les ailettes 45 sont dimensionnées pour former des surfaces d'appui pour l'index et le majeur de l'utilisateur (schématisés par des cercles en traits interrompus sur la figure 5), et sont raccordées l'une à l'autre par des portions intermédiaires 50 de la bague 6.

La jupe 46 définit un logement propre à recevoir étroitement, en fin d'injection, la tête 17, comme le montrent les figures 10 et 11. Elle présente deux fenêtres 51 diamétralement opposées et un chanfrein proximal 52, visible notamment sur la figure 11.

Comme cela apparaît sur les figures 10 et 11, la bague 6 est destinée à être engagée sur l'extrémité proximale de l'étui 5 jusqu'au-delà des dents 33, ce qui la verrouille définitivement sur la portion 26 de l'étui 5. Le chanfrein 52 est quant à lui destiné à permettre l'engagement de la tête 17 dans le logement défini par la paroi 46, jusqu'à encliquetage, en fin d'utilisation, du rebord 24 derrière des nervures 53 aménagées sur les zones de la bague 6 qui délimitent les bords proximaux des fenêtres 51. Cet encliquetage assure le verrouillage de la tige 4 dans la position montrée sur les figures 10 et 11. La portion 17a de la tête 17 assure le maintien des dents 33 dans une position radiale extérieure, qui permet d'assurer le verrouillage de la bague 6.

Les parties de maintien 47 sont diamétralement opposées et présentent chacune une forme de fourche, c'est-à-dire comprennent chacune une paire de bras 55 et un corps de liaison 56 reliant ces bras 55 à la bague 6.

Les bras 55 sont conformés, notamment arrondis dans l'exemple représenté, de manière à pouvoir entourer partiellement le corps de seringue 2, et comprennent des chanfreins 57 aménagés sur leur côté proximal, par lequel un corps de seringue 2 est destiné à être introduit au travers de la bague 6.

Chaque bras 55 comprend également, au niveau de son extrémité libre, un bossage 58 faisant saillie radialement vers l'intérieur. Il peut comprendre plusieurs de tels bossages.

Le corps de liaison 56 de chaque partie de maintien 47 présente une forme coudée, c'est-à-dire comprend une partie de base, reliée au reste de la bague 6 et disposée sensiblement parallèlement à l'axe de cette bague 6, et une partie intermédiaire, reliée auxdits bras 55 et disposée sensiblement perpendiculairement à l'axe de la bague 6.

Les parties de base sont disposées sur le même diamètre que celui selon lequel font saillie les ailettes 45.

Ainsi que le montre la figure 5, les corps de liaison 56 sont conformés de telle sorte qu'après assemblage de la bague 6 sur l'étui 5, lesdites parties intermédiaires sont engagées à coulissement dans les saignées 32 et que lesdites parties de base font saillie par rapport à la paroi de l'étui 5, grâce aux méplats 26a de la portion 26.

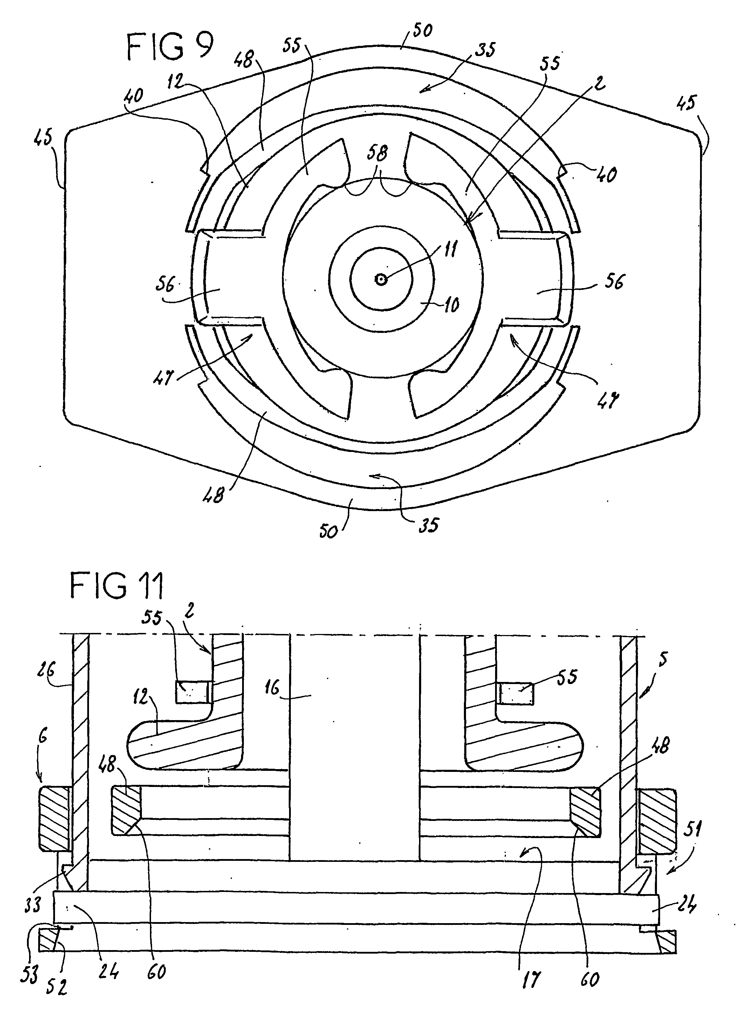

Comme cela se comprend en référence aux figures 8 et 9, les bras 55 sont destinés à recevoir entre eux et à enserrer le corps de seringue 2, et peuvent être déformés élastiquement de manière à pouvoir recevoir entre eux, et enserrer, des corps de seringue 2 de diamètres extérieurs différents, en particulier un corps 2 dit "0,5 ml" comme montré sur la figure 8 et un corps 2 dit "1 ml" comme montré sur la figure 9.

Les chanfreins 57 permettent de faciliter l'engagement d'un corps de seringue 2 entre ces bras, et les bossages 58 permettent d'assurer l'immobilisation et le centrage d'un corps de seringue 2 de diamètre réduit.

La relative souplesse des corps de liaison 56 dans le sens radial de la bague 6 permet de placer les bras 55 selon plusieurs positions radiales distinctes, et donc d'assurer un bon serrage d'un corps de seringue 2, sur une large gamme de diamètres de ceux-ci.

Les pattes 30 et les bossages 31 permettent quant à eux, ainsi que cela se déduit de la figure 4, de caler latéralement des corps de seringue 2 de diamètres extérieurs différents, les pattes 30 se déformant au besoin vers l'extérieur de manière à permettre l'effacement adéquat des bossages 31.

En outre, la présence desdites parties de base des corps de liaison 56 permet d'écarter les doigts de l'utilisateur par rapport à la paroi de l'étui 5, comme le montre la figure 5, éliminant ainsi tout risque de friction susceptible de freiner le mouvement d'activation du système de verrouillage, voire de compromettre ce verrouillage.

Les deux parties d'assemblage 48 sont délimitées par l'ouverture centrale de la bague 6 d'une part et par les lumières 35 d'autre part. Elles ont une épaisseur telle qu'elles sont déformables élastiquement dans le sens radial, cette déformabilité étant rendue importante grâce aux fentes 41.

Ces parties 48 constituent par exemple un anneau de forme ovale, relié, au niveau de leurs zones périphériques, les plus éloignées l'une de l'autre, à la bague 6, à proximité des parties de base des corps de liaison 56. Ces zones périphériques sont situées l'une par rapport à l'autre à une distance supérieure au diamètre externe de la collerette 12 du corps 2 tandis que les zones médianes de cet anneau, les plus proches l'une de l'autre, sont situées l'une par rapport à l'autre, à l'état non déformé de l'anneau, à une distance inférieure au diamètre extérieur de cette collerette 12.

La figure 11 montre que la partie proximale 26 de l'étui 5 présente un diamètre tel qu'il existe un espace entre la paroi de cette partie proximale 26 et lesdites zones médianes dudit anneau, cet espace permettant la déformation radiale de ces zones médianes.

En outre, des chanfreins 60 sont aménagés dans ces mêmes zones médianes.

Ainsi que cela se déduit des figures 6, 8, 9 et 11, la collerette 12 est destinée à être engagée au travers de l'anneau ovale précité jusqu'au-delà des parties 48. Les chanfreins 60 de ces parties 48 permettent l'effacement desdites zones médianes pour autoriser le passage de la collerette 12. Le rappel élastique de ces parties 48 dans leur forme d'origine permet le verrouillage de la collerette 12 au-delà de ces parties 48.

Une autre forme de réalisation peut consister en ce que les parties d'assemblage 48 constituent un anneau sensiblement circulaire avec un ou plusieurs bossages dans les zones médianes, les plus déformables.

Le corps 2 est alors parfaitement assemblé à la bague 6, sans aucun jeu radial et avec un jeu axial très limité.

En pratique, comme cela se comprend par comparaison des figures 2 et 10, l'utilisateur réalise l'injection en exerçant des pressions antagonistes sur les ailettes 45 et sur la tête 17, au moyen respectivement de son majeur et de son index, d'une part, et de son pouce, d'autre part, selon le geste classique précité.

A la fin de l'injection, le piston 3 vient en butée contre le fond du corps de seringue 2 ou la tête 17 vient en butée contre l'étui 5 ; lesdites forces antagonistes peuvent alors être exercées de manière plus intense, jusqu'à rupture des ponts 20. Il en résulte un déplacement relatif brusque de la bague 6, et donc du corps de seringue 2, par rapport à la tête 17, jusqu'à venue de la bague 6 et de la tête 17 dans une position de sécurité, montrée sur la figure 10, dans laquelle l'aiguille 11 est complètement rétractée dans l'étui 5.

Ce mouvement brusque est réalisé dans le prolongement du geste d'utilisation de la seringue 1 pour l'injection. Le caractère brusque de ce mouvement, associé à l'écartement des doigts de l'utilisateur par rapport à l'étui 5 résultant de la présence desdites parties de base des corps de liaison 56, assure que la position de sécurité est effectivement atteinte dans tous les cas.

Le verrouillage de la tête 17 est réalisé automatiquement en fin de ce mouvement brusque, par engagement du rebord 24 derrière les nervures 53. L'engagement de la portion 17a de la tête 17 dans l'extrémité proximale de l'étui 5, comme le montre la figure 11, élimine, ou tout au moins rend très difficile, toute tentative de déverrouillage de la tige 4.

Ainsi qu'il apparaît de ce qui précède, l'invention fournit une seringue ayant pour avantage essentiel de pouvoir recevoir des corps de seringue de différents diamètres, de sorte que cette seringue peut comprendre des pièces standardisées et recevoir des corps de seringue différents, en fonction des besoins.

Il va de soi que l'invention n'est pas limitée à la forme de réalisation décrite ci-dessus à titre d'exemple mais qu'elle en embrasse au contraire toutes les variantes de réalisation couvertes par les revendications ci-annexées.

## Revendications

1. Seringue, comprenant un corps de seringue (2) et une bague (6) à laquelle le corps de seringue (2) est relié du côté proximal ;
seringue (1) **caractérisée en ce que** :
- la bague (6) comporte deux parties de maintien (47) destinées à recevoir et à maintenir entre elles le corps de seringue (2) ; chaque partie de maintien (47) présente une forme de fourche, c'est-à-dire comprend une paire de bras (55) et un corps de liaison (56) reliant ces bras (55) à la bague (6), les bras (55) de chaque partie de maintien (47) étant conformés de manière à pouvoir entourer partiellement le corps de seringue (2) ; et
- lesdits bras (55) sont réalisés en un matériau présentant un degré de souplesse élastique tel qu'ils peuvent être déformés élastiquement de manière à pouvoir recevoir entre eux, et enserrer, des corps de seringue (2) de diamètres extérieurs différents.

2. Seringue selon la revendication 1, **caractérisée en ce que** les parties de maintien (47) sont diamétralement opposées.

3. Seringue selon la revendication 1 ou la revendication 2, **caractérisée en ce que** chacun des bras (55) d'une partie de maintien (47) comprend au moins un bossage (58) faisant saillie radialement vers l'intérieur.

4. Seringue selon l'une des revendications 1 à 3, **caractérisée en ce qu'**un chanfrein (57) est aménagé dans lesdits bras (55) du côté de la bague (6) par lequel un corps de seringue (2) est destiné à être introduit au travers de cette bague (6).

5. Seringue selon l'une des revendications 1 à 4, **caractérisée en ce qu'**elle comprend un étui de protection (5), engagé autour du corps de seringue (2), cet étui (5) et ce corps (2) étant mobiles l'un par rapport à l'autre entre une position d'utilisation de la seringue (1), dans laquelle l'aiguille (11) de la seringue est exposée, et une position de sécurité, dans laquelle cette aiguille (11) est entourée par l'étui (5) de manière à prévenir tout risque de piqûre ou de coupure, et donc de contamination éventuelle, de l'utilisateur, après utilisation ; l'étui de protection (5) comprend au moins une lumière ou saignée longitudinale (32) ;
- la bague (6) forme un moyen de déplacement dudit corps (2) et dudit étui (5) l'un par rapport à l'autre entre lesdites positions d'utilisation et de sécurité, et présente à cet effet une partie logée à l'intérieur de l'étui de protection (5), comportant lesdites parties de maintien (47), et au moins une partie de prise d'appui (45), engagée au travers de ladite lumière ou saignée (32) et faisant saillie au-delà de l'étui de protection (5), qui sert à la prise d'appui de l'index ou du majeur de la main de l'utilisateur ;
- la tige de piston (4) de la seringue (1) comprend deux parties télescopiques (15, 16), dont une, distale, est reliée au piston (3) de la seringue, et dont l'autre, proximale, comporte une tête (17) formant une partie de prise d'appui pour le pouce de l'utilisateur ; et
- au moins un pont ruptible (20) relie les deux parties télescopiques (15, 16) de la tige de piston (4), ce ou ces ponts (20) étant propres à se rompre au-delà d'un seuil de forces antagonistes exercées sur ladite partie de prise d'appui (45) et ladite tête (17), ce seuil étant supérieur à la force nécessaire au déplacement du piston (3) mais inférieur aux forces antagonistes qu'un utilisateur est susceptible d'exercer manuellement sur ladite partie de prise d'appui (45) et ladite tête (17).

6. Seringue selon la revendication 5, **caractérisée en ce que** l'étui de protection (5) présente des moyens de calage (30, 31) permettant de caler latéralement un corps de seringue (2) mis en place dans cet étui (5), ces moyens de calage (30, 31) étant mobiles radialement de manière à pouvoir caler des corps de seringue (2) de diamètres extérieurs différents.

7. Seringue selon la revendication 6, **caractérisée en ce que** les moyens de calage (30, 31) sont aménagés du côté distal de l'étui de protection (5).

8. Seringue selon la revendication 6 ou la revendication 7, **caractérisée en ce que** l'étui de protection (5) présente au moins deux fentes en forme de "U" diamétralement opposées, qui individualisent chacune une patte (30) mobile radialement, la face interne de chacune de ces pattes (30) présentant, du côté de l'extrémité de la patte (30) non reliée à l'étui de protection (5), un bossage (31) faisant saillie à l'intérieur de cet étui (5).

9. Seringue selon l'une des revendications 1 à 8, **caractérisée en ce que** le corps de liaison (56) que comprend chaque partie de maintien (47) est réalisé en un matériau présentant un degré de souplesse élastique, de telle sorte que ce corps de liaison (56) présente une souplesse dans le sens radial de la bague (6), cette souplesse étant telle qu'elle permet de placer les bras (55) de la partie de maintien (47) selon plusieurs positions radiales distinctes.

10. Seringue selon l'une des revendications 1 à 9, **caractérisée en ce que** chaque corps de liaison (56) présente une forme coudée, c'est-à-dire comprend une partie intermédiaire, reliée auxdits bras (55) et disposée sensiblement perpendiculairement à l'axe de la bague (6), et une partie de base, reliée à la bague (6) et disposée sensiblement parallèlement à l'axe de cette bague (6).

11. Seringue selon l'une des revendications 5 à 10, **caractérisée en ce que** :
- la bague (6) comprend deux saillies (45) de prise d'appui, diamétralement opposées ;
- le corps de liaison (56) de l'une des parties de maintien (47) est reliée à la bague (6) à proximité de l'une de ces saillies de prise d'appui (45) et le corps de liaison (56) de l'autre partie de maintien (47) est relié à la bague (6) à proximité de l'autre de ces saillies (45), et
- au moins une partie de ces corps de liaison (56) est conformée de manière à faire saillie au-delà de la paroi de l'étui de protection (5).

## Patentansprüche

1. Spritze, die einen Spritzenkörper (2) und eine Ringstruktur (6) aufweist, mit der der Spritzenkörper mit der proximalen Seite verbunden ist, wobei die Spritze (1) **dadurch gekennzeichnet ist, dass**:
- die Ringstruktur (6) zwei Halteteile (47) aufweist, die dazu vorgesehen sind, den Spritzenkörper (2) aufzunehmen und zu halten, wobei jedes Halteteil (47) eine Gabelform, d.h. ein Paar von Armen (55), und ein Verbindungsstück (56) aufweist, das die Arme (55) mit der Ringstruktur (6) verbindet, wobei die Arme (55) eines jeden Halteteils (47) derart ausgestaltet sind, dass diese den Spritzenkörper (2) teilweise umschließen können, und
- die Arme (55) ein Material aufweisen, das ein Maß an elastischer Flexibilität hat, so dass diese derart elastisch deformiert werden können, um dazwischen Spritzenkörper (2) von unterschiedlichen Außendurchmessern aufzunehmen und zu umschließen.

2. Spritze nach Anspruch 1, **dadurch gekennzeichnet, dass** die Halteteile (47) diametral entgegengesetzt angeordnet sind.

3. Spritze nach Anspruch 1 oder 2, **dadurch gekennzeichnet, dass** jeder Arm (55) eines Halteteils (47) zumindest einen Vorsprung (58) aufweist, der radial nach innen gerichtet ausgebildet ist.

4. Spritze nach einem der Ansprüche 1 bis 3, **dadurch gekennzeichnet, dass** eine Fase (57) an den Armen (55) der Ringstruktur (6) angebracht ist, über die ein Spritzenkörper (2) durch die Ringstruktur (6) eingeführt werden kann.

5. Spritze nach einem der Ansprüche 1 bis 4, **dadurch gekennzeichnet, dass** diese eine Schutzhülle (5) aufweist, die um den Spritzenkörper (2) herum eingreifend angeordnet ist, wobei die Hülle (5) und der Körper (2) gegeneinander von einer Position zur Verwendung der Spritze (1), in der die Nadel (11) der Spritze exponiert ist, in eine Sicherheitsposition, in der die Nadel (11) von der Hülle (5) derart umschlossen ist, dass jegliches Stech- und Schneidrisiko und folglich jegliche eventuelle Kontamination des Benutzers nach der Benutzung verhindert wird, bewegt werden können, wobei die Schutzhülle (5) zumindest eine Längsöffnung oder einen Längseinschnitt (32) aufweist;
- die Ringstruktur (6) bildet ein Mittel zum verschieben des Körpers (2) in Bezug auf die Hülle (5) von der Verwendungs- in die Sicherheitsposition und weist dazu ein im Inneren der Schutzhülle (5) angeordnetes Teil auf, das die Halteteile (47) aufweist, und zumindest ein Haltegriffteil (45), das durch die Öffnung oder den Einschnitt (32) hindurchgreift und über die Schutzhülle (5) hinaus einen Vorsprung bildet, der dem Zeigefinger oder Mittelfinger der Hand des Benutzers als Haltegriff dient;
- die Kolbenstange (4) der Spritze (1) weist zwei Teleskopteile (15, 16) auf, von denen eines distal mit dem Kolben (3) der Spritze verbunden ist, und das andere proximal einen Kopf (17) aufweist, der ein Haltegriffteil für den Daumen des Benutzers ausbildet; und
- zumindest einen brechbaren Steg (20), der die beiden Teleskopteile (15, 16) der Kolbenstange (4) miteinander verbindet, wobei der oder die Stege (20) geeignet sind, um bei einer Gegenkraft, die über eine Schwelle hinausgeht, zu brechen, die auf das Haltegriffteil (45) und den Kopf (17) ausgeübt wird, wobei die Schwelle oberhalb der Kraft liegt, die zur Verschiebung des Kolbens (3) erforderlich ist, aber unterhalb der Gegenkräfte liegt, die von einer Bedienperson manuell auf das Haltegriffteil (45) und den Kopf (17) ausgeübt werden.

6. Spritze nach Anspruch 5, **dadurch gekennzeichnet, dass** die Schutzhülle (5) Klemmmittel (30, 31) aufweist, die ein seitliches Festklemmen eines in der Schutzhülle (5) platzierten Spritzenkörpers (2) ermöglichen, wobei die Klemmmittel (30, 31) radial beweglich sind, so dass diese Spritzenkörper (2) von verschiedenen Außendurchmessern festklemmen können.

7. Spritze nach Anspruch 6, **dadurch gekennzeichnet, dass** die Klemmmittel (30, 31) an der distalen Seite der Schutzhülle (5) angebracht sind.

8. Spritze nach Anspruch 6 oder Anspruch 7, **dadurch gekennzeichnet, dass** die Schutzhülle (5) zumindest zwei diametral entgegengesetzt angeordnete Spalten in Form eines "U" aufweist, die jeweils eine radial bewegliche Zunge (30) individualisieren, wobei die Innenfläche einer jeden Zunge (30) an der am Ende liegenden Seite der Zunge (30), die nicht mit der Schutzhülle (5) verbunden ist, einen Vorsprung (31) aufweist, der sich in das Innere der Hülle (5) erstreckt.

9. Spritze nach einem der Ansprüche 1 bis 8, **dadurch gekennzeichnet, dass** das Verbindungsstück (56), das jedes der Halteteile (47) aufweist, ein Material aufweist, das ein Maß an elastischer Flexibilität hat, so dass das Verbindungsstück (56) eine Flexibilität in radialer Richtung der Ringstruktur (6) hat, wobei es diese Flexibilität erlaubt, dass die Arme (55) der Halteteile (47) in verschiedene radiale Positionen gebracht werden können.

10. Spritze nach einem der Ansprüche 1 bis 9, **dadurch gekennzeichnet, dass** jedes Verbindungsstück (56) eine gebogene Form hat, d.h. ein Zwischenteil, das mit den Armen (55) verbunden ist und im Wesentlichen rechtwinklig zu der Achse der Ringstruktur (6) angeordnet ist, und ein Basisteil aufweist, das mit der Ringstruktur (6) verbunden ist und im Wesentlichen parallel zu der Achse der Ringstruktur (6) angeordnet ist.

11. Spritze nach einem der Ansprüche 5 bis 10, **dadurch gekennzeichnet, dass**:
- die Ringstruktur zwei diametral entgegengesetzt angeordnete Haltegriffvorsprünge (45) aufweist;
- das Verbindungsstück (56) des einen Halteteils (47) mit der Ringstruktur (6) in der Nähe seines einen Haltegriffvorsprungs (45) verbunden ist, und das Verbindungsstück (56) des anderen Halteteils (47) mit der Ringstruktur (6) in der Nähe seinen anderen Vorsprunges (45) verbunden ist, und
- zumindest ein Teil des Verbindungsstücks (56) derart ausgebildet ist, dass der Vorsprung über die Wand der Schutzhülle (5) hinausgeht.

## Claims

1. A syringe, comprising a syringe body (2) and a ring (6) to which the syringe body (2) is connected at the proximal end ;
in which syringe (1) :
- the ring (6) has two holder parts (47) intended to receive and to hold the syringe body (2) between them; each holder part (47) has a fork shape, that is to say comprises a pair of arms (55) and a connection body (56) connecting these arms (55) to the ring (6), the arms (55) of each holder part (47) being configured in such a way as to be able to partially enclose the syringe body (2); and
- said arms (55) are made of a material having a degree of elastic flexibility which is such that they can be elastically deformed in order to be able to receive between them, and tightly grip, syringe bodies (2) of different external diameters.

2. The syringe as claimed in claim 1, wherein the holder parts (47) are diametrically opposed.

3. The syringe as claimed in claim 1 or claim 2, wherein each of the arms (55) of a holder part (47) comprises at least one boss (58) projecting radially inward.

4. The syringe as claimed in one of claims 1 through 3, wherein a bevel (57) is arranged in said arms (55) on the side of the ring (6) via which a syringe body (2) is intended to be introduced through this ring (6).

5. The syringe as claimed in one of claims 1 through 4, wherein it comprises a protective casing (5) engaged around the syringe body (2), this casing (5) and this body (2) being movable relative to one another between a use position of the syringe (1), in which the needle (11) of the syringe is exposed, and a safety position, in which this needle (11) is surrounded by the casing (5), in such a way as to prevent any risk of stick injuries or cuts, and thus of possible contamination of the user, after use; the protective casing (5) comprises at least one longitudinal slot or cutting (32) ;
- the ring (6) forms a means of displacing said body (2) and said casing (5) relative to one another between said use position and safety position, and for this purpose it has a part lodged inside the protective casing (5), including said holder parts (47), and at least one support part (45), engaged through said slot or cutting (32) and protruding beyond the protective casing (5), and which serves to support the index finger or middle finger of the user's hand;
- the plunger rod (4) of the syringe (1) comprises two telescopic parts (15, 16), one of which, the distal part, is connected to the plunger (3) of the syringe, and the other of which, the proximal part, has a head (17) forming a support part for the user's thumb; and
- at least one breakable bridge (20) connects the two telescopic parts (15, 16) of the plunger rod (4), said bridge or bridges (20) being able to break above a threshold of antagonistic forces exerted on said support part (45) and said head (17), this threshold being greater than the force needed to displace the plunger (3) but lower than the antagonistic forces which a user is likely to exert manually on said support part (45) and said head (17).

6. The syringe as claimed in claim 5, wherein the protective casing (5) has wedge means (30, 31) with which it is possible to laterally wedge a syringe body (2) placed in this casing (5), these wedge means (30, 31) being radially movable in such a way as to be able to wedge syringe bodies (2) of different external diameters.

7. The syringe as claimed in claim 6, wherein the wedge means (30, 31) are arranged at the distal end of the protective casing (5).

8. The syringe as claimed in claim 6 or claim 7, wherein the protective casing (5) has at least two diametrically opposed U-shaped slits which each create a radially movable tab (30), the inner face of each of these tabs (30) having, toward the end of the tab (30) not connected to the protective casing (5), a boss (31) which protrudes toward the inside of this casing (5).

9. The syringe as claimed in one of claims 1 through 8. wherein the connection body (56) provided on each holder part (47) is made of a material having a degree of elastic flexibility which is such that this connection body (56) has a flexibility in the radial direction of the ring (6), this flexibility being such that it allows the arms (55) of the holder part (47) to be placed in a plurality of distinct radial positions.

10. The syringe as claimed in one of claims 1 through 9, wherein each connection body (56) has an elbowed configuration, that is to say comprises an intermediate part connected to said arms (55) and arranged substantially perpendicular to the axis of the ring (6), and a base part connected to the ring (6) and arranged substantially parallel to the axis of this ring (6).

11. The syringe as claimed in one of claims 5 through 10, wherein:
- the ring (6) comprises two diametrically opposed support projections (45);
- the connection body (56) of one of the holder parts (47) is connected to the ring (6) in proximity to one of these support projections (45), and the connection body (56) of the other holder part (47) is connected to the ring (6) in proximity to the other of these projections (45), and
- at least one part of these connection bodies (56) is configured in such a way as to project beyond the wall of the protective casing (5).
